(19) Europäisches Patentamt
European Patent Office
Office européen des brevets

(11) Publication number: **0 124 221**
**A1**

# (12) EUROPEAN PATENT APPLICATION

(21) Application number: 84301601.5

(22) Date of filing: 09.03.84

(51) Int. Cl.³: C 12 Q 1/68

(30) Priority: 09.03.83 GB 8306426

(43) Date of publication of application: 07.11.84
Bulletin 84/45

(84) Designated Contracting States: BE CH DE FR GB IT LI NL SE

(71) Applicant: Malcolm, Alan David Blair, 22 Elm Crescent, Ealing London, W5 3JW (GB)
Applicant: Nicolas, Jean Lesley, 102 Cherrywood Lane, Morden Surrey, SM4 4HB (GB)

(72) Inventor: Malcolm, Alan David Blair, 22 Elm Crescent, Ealing London, W5 3JW (GB)
Inventor: Nicolas, Jean Lesley, 102 Cherrywood Lane, Morden Surrey, SM4 4HB (GB)

(74) Representative: Lambert, Hugh Richmond et al, D. YOUNG & CO. 10 Staple Inn, London, WC1V 7RD (GB)

## (54) Method of detecting a polynucleotide sequence and labelled polynucleotides useful therein.

(57) A method of detecting nucleic acid sequences is disclosed and enzyme-labelled reagents for use therein. According to the invention sample polynucleotide is first hybridised with a polynucleotide probe comprising a nucleic acid sequence complementary to that which is to be detected, and having a polynucleotide tail, preferably a single stranded poly(dA) or poly(dT) tail. Following attachment of the probe to the sample, an enzyme-containing marker, is attached to the probe, the marker comprising a polynucleotide having a single-stranded portion, to the base groups of which is or are attached one or more enzymatically active groups, and a polynucleotide tail which can be attached to the probe by annealing or hybridisation. Preferably the marker has a single-stranded polynucleotide tail complementary to that on the probe whereby the marker can be attached to the probe by a second hybridisation step. Presence of the sequence to be detected is confirmed by testing the labelled sample for any enzyme related activity.

EP 0 124 221 A1

# Method of Detecting a Polynucleotide Sequence and Labelled Polynucleotides useful therein

## Field of the invention

This invention is in the fields of medical diagnostics and biotechnology. It relates to a method of detecting gene sequences. It is capable of distinguishing between sequences differing by only one base-pair. It will therefore be useful in the detection of genetic disorders, and viral infection and in the identification of bacteria with regard to their antibiotic resistance. It includes labelled polynucleotides useful in the method and diagnostic kits containing labelled polynucleotides.

## Description of prior art

Many procedures employed in biomedical research and recombinant DNA technology rely heavily on the use of polynucleotides radioactively labelled, usually with isotopes of hydrogen ($^{3}H$), phosphorus ($^{32}P$), carbon ($^{14}C$), or iodine ($^{125}I$). Such radioactive compounds provide useful indicator probes that permit the user to detect, monitor, localize, or isolate nucleic acids and other molecules of scientific or clinical interest, even when present in only extremely small amounts. To date, radioactive materials have provided the most sensitive, and in many cases the only, means to perform many important analytical tests. There are, however, serious limitation and drawbacks associated with the use of radioactive compounds. First, since personnel who handle radioactive material can be exposed to potentially hazardous levels of radiation, elaborate safety precautions must be maintained during the preparation, use and disposal of the radiostopes. Secondly, radioactive nucleotides are extremely expensive to buy and use, in large part due to the cost of equipment and manpower necessary to provide the appropriate safeguards, producer/user health monitoring services, and waste-disposal programs. Thirdly, in order to achieve the necessary sensitivity, radioactive materials of high specific

activity must be used. This implies a correspondingly short half-life and therefore a limited shelf-life, which further increases costs.

In view of the above problems encountered with radiolabelling, other kinds of label have been tried. Thus D.C. Ward and his colleagues at Yale University attached biotin groups chemically to deoxyribonucleotides and introduced these nucleotides into DNA by nick translation. From this biotin-labelled ds-DNA they prepared single-stranded DNA having a sequence complementary to a DNA sequence to be detected, and contacted the resultant biotin labelled probe DNA with the DNA sequence to be detected under hybridisation conditions. To detect hybridisation use was made of a biotin/anti-biotin, biotin/avidin or similar interaction. The avidin or anti-biotin is then labelled, e.g. by fluorescence or enzymatically. See European Patent Specification 63879 (Yale University), P.R. Langer, A.A. Waldrop and D.C. Ward, Proc. Natl. Acad.Sci USA 79 6633-6637 (1981) and Singer and D.C. Ward, ibid 79 3331-7335 (1982). One problem with this method is that the number of biotin groups which can be introduced is limited. It is quoted in the Patent Specification as from 10-100 biotin groups per kilobase. At all events, the preferred method of labelling involves amplifying the signal. Rabbit anti-biotin IgG is coupled to from 15 to 20 hapten (DNF) groups. An antibody is then raised to the DNP. This antibody can be biotin-labelled and the "sandwich" repeated. The final IgG is fluorescently or enzymatically labelled. Another problem with this method is that each individual probe appropriate to detecting one sequence is modified to introduce the biotin groups, and thence the label. In other words, labelling is specific to the end-use and not "universal".

In GB-A- 2 019 408 an allegedly general technique is disclosed for the detection of a given nucleotide sequence in a polynucleotide and which comprises hybridizing the polynucleotides with a probe comprising a nucleic acid sequence complementary to the sequence to be detected and in which the probe is "chemically modified" for coupling with an enzyme, the subsequent attachment and detection of which is used to indicate the presence, or absence, of the given sequence in the original sample. In

practice, the only method disclosed for the chemical modification of the probe and attachment of the enzyme is via an avidin/biotin complex; thus in terms of its technical teaching the method of GB-A-2 019 408 is the same as that proposed by Ward et al in EP-A-0063879.

Although the technique disclosed in GB-A-2 019 408 is allegedly a universal technique involving a two-stage marking of the sample, e.g. initial hybridisation of the sample with the chemically modified probe, i.e. the biotinised polynucleotide, followed by attachment of the enzyme in the form of an avidin/enzyme complex, in practice the method is extremely difficult to carry out with any degree of success and the results obtained are extremely variable. The method is therefore extremely unreliable and does not provide any basis for polynucleotide sequence detection to be carried out as a matter of routine

The mapping of genes in chromosomes is another field in which DNA sequences are detected. Recent papers include a review by F.H. Ruddle, Nature, 294 115 (1981) and a description of gene mapping of *Drosophila* chromosomes, by M. Wu and N. Davidson, Proc. Natl. Acad. Sci.USA 78 7059-7063 (1981). Spherical particles of gold, visible by electron microscopy, were prepared as a colloidal sol. Bovine serum albumin was absorbed onto their surfaces. To crosslink DNA to the BSA-coated particles, photoactivable nitrene groups were covalently bonded, by amide bonds, to the groups of the BSA. Single-stranded DNA was attracted electrostatically to the gold particles, which at pH 4 are positively charged. By photolysis of the nitrene groups the DNA was covalently linked to the BSA and thence to the gold spheres. Poly(dT) tails were added to the DNA by terminal deoxynucleotidyl transferase (hereinafter called "TdT")-catalysed addition of thymidine nucleotides. Separately, cloned double-stranded DNA containing a *Drosophila* gene sequence was prepared, 3'-end poly (dA)-tailed and denatured to single-stranded DNA. This poly(dA)-tailed "stock DNA" was hybridised to *Drosophila* polytene chromosomes on a glass slide. Finally, the resultant polytene chromosome/poly(dA)-tailed stock DNA duplex was hybridised at its poly(dA) tail to the poly (dT) tail of the gold sphere-labelled

DNA. The squash of Drosophila polytene chromosomes, bearing their gold sphere label, was then studied under the electron microscope.

Steps towards the linking of DNA to a protein capable of binding to a specific receptor on the surface of a cell have been taken by S. Cheng, G.T. Merlino and I.H.Pastan, Nucleic Acids Research 11 659-668. These authors have linked the alpha (2)-macroglobulin protein to DNA by the steps of (1) tailing the DNA with dG, (2) reacting unpaired guanine bases with N-acetyl N'-(p-glyoxylylbenzoyl) cystamine in the presence of borate, thus generating a side-chain of formula:

$$-CO-NH-(CH_2)_2-S-S-(CH_2)_2-NHCOCH_3,$$

(3) reductively cleaving the S-S bond with dithiothreitol, to geneate a side-chain having a sulphydryl group, of formula:

$$-CO-NH-(CH_2)_2-SH$$

(4) making a derivative of the alpha (2)-macroglobulin and reacting it with the sulphydryl group-bearing DNA.

EP-A-0 070 685 (Standard Oil Company), published 26 January 1983, outlines a method of assaying a polynucleotide sequence, for example in a gene, in which the sample polynucleotide, in ss-form, is contacted under hybridisation conditions with two polynucleotide reagents complementary to different portions of the sample polynucleotide. The first reagent has attached to it a catalyst for chemiluminescent reaction and the second reagent has an absorber/emitter, whereby it absorbs light produced from a chemiluminescent reaction and emits light of longer wavelength. It is suggested that the catalyst could be peroxidase, to catalyse the oxidation of luminol by hydrogen peroxide, and the absorber/emitter could be porphyrin. The sample polynucleotide, with the first and second polynucleotide reagents (hybridised thereto when the sample is indeed of the suspected sequence), is then contacted with the chemiluminescent reagent, e.g.

luminol/$H_2O_2$, and the emitted light is measured. This patent specification is an unusual document, containing no working examples. Page 14 suggests that the chemiluminescent catalyst, such as peroxidase, should be coupled to the 5'-end of the first reagent by means of a short oligonucleotide linker segment. The segment would contain an aminohexane-adenosine nucleus at its 5' terminus followed by a short sequence of 4 to 6 adenosine or thymidine nucleotides. The linker segment is to be attached to the 5'-terminus of the reagent through "the appropriate use of basic ligation reactions".

Another assay employing a polynucleotide reagent labelled with a "light label" such as a chemiluminescent catalyst. e.g. peroxidase or luciferase, is proposed in European Patent Specification 70687 (Standard Oil Company), which gives even less information as to how to attach the chemiluminescent catalyst to the reagent. It is said, vaguely, on page 6 that the light labels can be attached to any point on the reagent, but that terminal positions are more desirable. There is no further information, i.e. no working example and no prior art reference, explaining what is intended.

## Summary of the invention

The present invention is based on the finding that enzymes can be linked by wholly chemical bonds to the base groups of single-stranded polynucleotides and that the resulting pendant enzyme groups retain considerable enzymatic activity. Moreover, the enzyme-labelled single-stranded polynucleotide can be provided with means whereby it can be readily attached to a variety of other polynucleotides, especially to a polynucleotide containing a single-stranded sequence of nucleotides complementary to a polynucleotide sequence to be detected. For example, suppose it is desired to detect whether a clinical specimen contains an abnormal sequence "W" of DNA. DNA fragments, produced by restriction endonuclease digestion, are contacted under hybridisation conditions with a polynucleotide probe containing a polynucleotide sequence "c W" complementary to "W" and provided with a poly(dT) or poly (dA) tail. A "marker" polynucleotide, which can be any conveniently obtainable single-stranded

DNA, is enzyme-labelled, preferably with horseradish peroxidase, and is given a poly (dA) or poly (dT) tail. The polynucleotide probe and the enzyme-labelled marker polynucleotide are hybridised, whereby poly (dA) or (dT) sequences in the tail of the enzyme-labelled marker polynucleotide hybridise to the poly (dT) or (dA) sequences respectively, in the tail of the polynucleotide probe. If the DNA sequence "W" to be detected was indeed present in the specimen, "W" will have hybridised to the complementary sequence " c W" of the probe. Since the probe is hybridised to the marker polynucleotide and since the marker polynucleotide is enzyme-labelled, the hybridisation of "W" will be indicated by enzymatic activity being found in the washed product.

Accordingly, the present invention provides:

(1) a polynucleotide (hereinafter called "a marker or enzyme-labelled polynucleotide") comprising a single-stranded portion in which enzymatically active groups are chemically linked to the base groups of the single-stranded portion of the polynucleotide";

(2) a marker polynucleotide as defined under (1) further comprising a polynucleotide tail, preferably a single-stranded polynucleotide tail whereby it may be attached, e.g. by hybridisation or annealing, to a sequence which will usually be a complementary sequence on a second polynucleotide (hereinafter called a "polynucleotide probe");

(3) a method of detection of a polynucleotide sequence, especially of a DNA or RNA sequence, and most especially of a DNA sequence derived from a clinical specimen, the presence or absence of which in a sample is suspected, which method comprises (a) contacting the sample with a polynucleotide probe comprising a single-stranded polynucleotide sequence complementary to the sequence to be detected under hybridisation conditions, (b) before or after said contact under hybridisation conditions attaching the polynucleotide probe to an enzyme-labelled marker polynucleotide as defined under (1) or (2), preferably by hybridisation between a

single stranded polynucleotide tail on the probe and a complementary single-stranded polynucleotide tail on the marker, and (c) detecting the presence or absence of enzyme-associated (hereinafter "enzymatic") activity in the sample; and

(4) a diagnostic kit comprising:

(a) a polynucleotide probe as defined under (3) above; and
(b) an enzyme-labelled marker polynucleotide as defined under (1) or (2).

The term "polynucleotide" is used herein to cover DNA, RNA and other connected nucleosides. In the context of the probe, factors relevant to the minimum chain length are the stability of the hybrid which is to be formed with the sequence to be detected and the probability of the sequence occuring in some other region of the genome (or other polynucleotide) than that which is under investigation. However, generally stated, the probe will have a chain of length at least 12-16 base-pairs (or bases of a single strand), excluding any polynucleotide tail sequences. A usually preferred range would be from 14 to 20. Thus the polynucleotide probe, before tailing, is typically an "oligonucleotide" (which term is included within the term "polynucleotide" for the purposes of this specification). The terms poly(dA), (dT), (dC), (dG), etc. applied to tails, cover any length of tail effective to bring about hybridisation with a complementary tail. Tail lengths of from 20 to 400 nucleotides are typical. The marker polynucleotide could in principle also have a short chain length, but ordinarily a length of at least 2.5 and more preferably at least 5 kilobases would be desirable. The minimum chain length depends mainly on the number of enzyme groups which can be incorporated and the sensitivity of the means ultimately used to detect the enzyme.

Detailed Description

Important preferred features of the invention as summarised above are as follows. The marker polynucleotide is provided with a single-strand tail of a homopolymer sequence, for example poly(dT), and the probe is provided with a complementary single-strand tail, for example poly(dA), whereby the attachment of the probe to the enzyme-labelled polynucleotide is by hybridisation. Complementary heterogeneous tails can be used on the marker and the probe but obviously homogeneous tails are preferred. Other methods of attachment can be contemplated, including the use of sticky-ended ds-DNA tails and blunt-ended ligation.

In the method of detection it is preferred first to contact the sample with the polynucleotide probe defined under (2) above under hybridisation conditions, i.e. the contact is effective to bring about hybridisation if the sample does indeed contain the sequence to be detected. Subsequently the resultant duplex product is hybridised to the enzyme-labelled polynucleotide. This order is at present preferred because the first-mentioned hybridisation normally requires more stringent conditions, which might effect the enzyme label adversely. However, the method as defined under (3) above covers the alternative possibility of first hybridising (or otherwise linking) the polynucleotide probe defined under (2) to the enzyme-labelled polynucleotide, to form an enzyme-labelled probe and thereafter contacting this enzyme-labelled probe with the sample under hybridisation conditions.

The invention is preferably practised using a glycoproteinaceous enzyme, e.g horseradish peroxidase, and linking it to amino groups in the base groups of the polynucleotide (especially at dA, dC or dG). The invention provides (5) a method of labelling a polynucleotide with a glycoproteinaceous enzyme, especially horseradish peroxidase, which method comprises oxidising the enzyme to convert cis-glycol linkages to aldehyde groups, reacting the oxidised enzyme with the polynucleotide to form Schiffs base linkages (-N=CH-) between amine groups of nucleotides and aldehyde groups of the oxidised enzyme, and reducing the Schiffs base linkages (to yield $-NH-CH_2-$or $N-CH_2-$linkages). Reduction can be carried out with a borohydride or cyanoborohydride reducing agent.

The complete reaction sequence is as follows:

REACTION SCHEME A

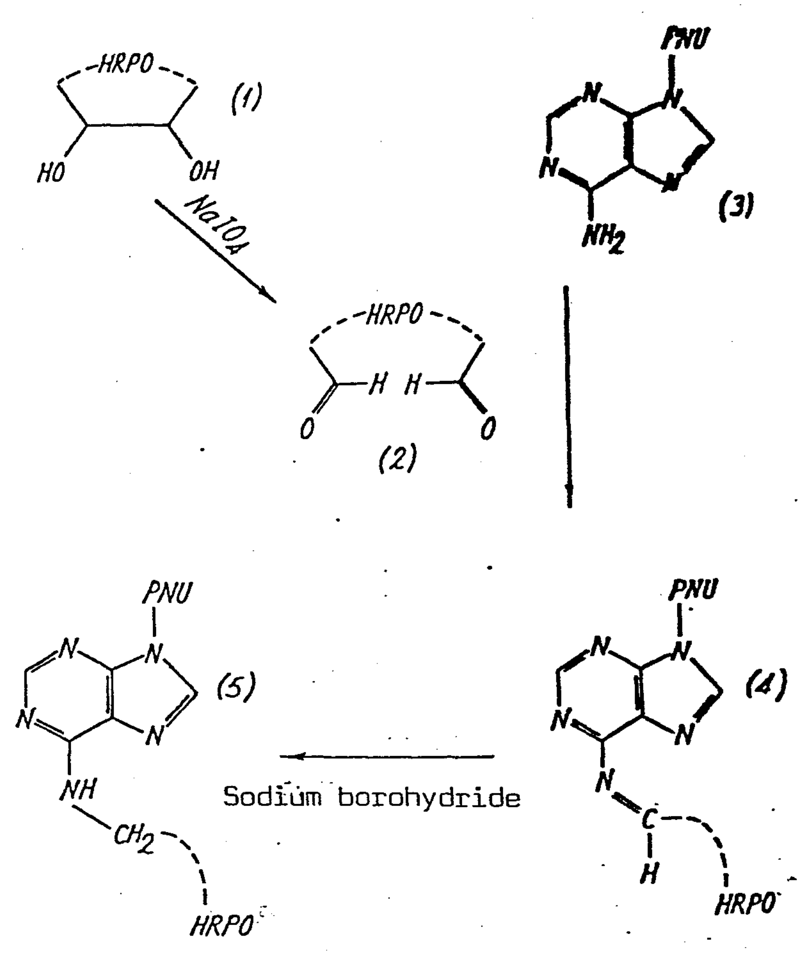


(1) (3) (2) (5) (4)

where formula (1) represents horseradish peroxidase (HRPO) and showing one of the glycollic groups therein and formula (3) represents a length of single-stranded polynucleotide (PNU) and showing one of the base groups of the nucleotide, in this case, and purely illustratively, an adenyl group, although it could equally well be a guanine or cytosine residue. Moreover, as will be seen from formula (2) the oxidation of the glycollic groups in the enzyme yields two aldehyde groups both of which can react with free amino groups in the nucleotide bases to form Schiff's bases which are subsequently reduced by reaction with sodium borohydride. Thus although the reaction scheme merely illustrates linking of the HRPO to one PNU base for the sake of clarity, the possibility exists for the HRPO to link to two PNU bases in the same or different PNU chains.

A similar method, but for attaching HRPO to antibodies (IgG or Fab), was described by M.B. Wilson and P.K. Nakane in "Immunofluorescene and related staining techniques", ed W. Knapp et al., publ. Elsevier/North Holland Biomedical Press 1978, pages 215-224. However, in heavily-labelled conjugates the antibody lost a great deal of specific antibody activity. It was ,therefore, uncertain whether the introduction of the enzyme would have an adverse effect on a DNA molecule, for example in terms of loss of enzyme activity after hybridisation, and it was also unpredictable whether a sufficient number of enzyme molecules could be attached to a DNA backbone to impart adequate enzyme activity to a probe, bearing in mind particularly that approach of a substrate to the enzyme might be expected to be highly sterically hindered. Fortunately, however, this enzyme-labelling procedure is successful. It is estimated that typically at least 5 and preferably at least 20, enzyme residues, especially HRPO ones, can be introduced per kilobase length of the marker polynucleotide. Possibly one enzyme residue could be linked to more than one molecule of polynucleotide.

In the present invention, the enzymes are attached via the nucleotide bases, in contrast to the suggestions about terminal attachment made in the above-mentioned European Patent Specifications 70685 and 70687.

The high degree of retention of enzyme activity and high degree of enzyme incorporation means that as small an amount as 10 attomoles ( 1 attomole = $10^{-18}$ mole) of a given sequence is detectable by the method of the invention. This is the quantity of a single copy gene which can be obtained by amniocentesis from a 16-week old foetus or 10 ml of blood from an adult. DNA can also be obtained from chorionic villi, see R. Williamson et al., The Lancet 2, 1125 (1981). All other applications of the method of the invention are likely to be less demanding on sensitivity.

The invention is of particular interest in the detection of particular sequences of a genome, especially in the diagnosis of disorders of human haemoglobin genes. A common disorder is sickle cell anaemia found predominantly in black people of West African descent. This genetic disorder consists of a point mutation in the beta-globin gene in which an adenine group is replaced by a thymine one, with the result that the defective gene containes the GTG codon for valine instead of the GAG codon for glutamate. A first step in identifying the disorder is to make a blot of appropriate DNA fragments. Thereafter, a labelled polynucleotide is contacted with the fragments under hybridisation conditions and the fragments then become labelled if hybridisation has taken place.

One method of preparing appropriate DNA fragments involves use of a restriction enzyme which acts on the normal beta-globin gene but not on the defective one. For example, the enzyme Dde I recognises the following underlined sequence in the normal gene but not the counterpart sequence of the defective gene:

| | | | |
|---|---|---|---|
| CCT | GAG | GAG | normal gene |
| CCT | GTG | GAG | defective gene |

Thus, fragments of the beta-globin gene are further restricted by Dde I, subjected to gel electrophoresis and transferred to a blotting paper, such as nitrocellulose or diazobenzyloxymethyl paper. At some stage the DNA is denatured to single-stranded form. Individuals having completely normal

beta-globin genes show cleavage of all the appropriate DNA into two smaller fragments, which can conveniently be called "V" and "W". Those having sickle cell trait, in which one beta-globin gene is normal and the allele has a defective beta-globin gene, show some "W", i.e. uncleaved, DNA, as well as V and W individually. Sickle cell indivduals give rise to no V or W, but wholly VW DNA. By appropriate use of probes complementary to the V fragments but not the W, and W fragments but not the V, these various conditions can be distinguished. Thus R.F. Geever et al., Proc. Natl. Acad. Sci. U.S.A., 78, 5081-5085 (1981) described one such method using radiolabelled probes.

Another method of producing appropriate DNA fragments has recently been described by B.J. Conner et al., Proc. Natl. Acad. Sci. U.S.A., 80, 278-282 (1983). DNA fragments from normal beta-globin genes were subjected to hybridisation conditions in the presence of radiolabelled synthetic single-stranded polynucleotide probes 19 nucleotides long, one matched, i.e. complementary to the normal gene and one mismatched, i.e. complementary to the defective gene. By choice of suitably stringent hybridisation conditions, it was found that the mismatched probe would not hybridise. Similarly, the normal probe would not hybridise to DNA fragments from defective genes.

Either of the above methods can be adapted for use in the present invention using the enzyme-labelled marker DNA and polynucleotide probe as described above in connection with the invention, in place of a radiolabelled probe.

The method of the invention is advantageous over the above-described method using biotin-labelled nucleotides, in that the probing sequence can be kept short, yet a highly "concentrated" label made by use of a long chain enzyme-labelled marker DNA for attachment to the probe.

The invention can also be used to detect DNA sequences in other kinds of cell than blood cells, in particular to detect mutated genes in

tumour cells. Besides medical diagnosis, the invention is applicable to diagnosis of genes present in bacterial cells, including antibiotic resistance genes, and to plant cell genes. It is also useful for detection of RNA sequences, for which purpose one could first prepare c DNA or could use a stock RNA containing a complementary sequence to that to be detected, comprising ribonucleotides. It is also envisaged that gene therapy techniques yet to be practised may involve the deletion of undesired RNA or DNA sequences and their replacement by synthetic polynucleotide sequences. The present invention would enable such polynucleotide sequences to be detected, thus confirming that the gene therapy has succeeded.

The HRPO residue could be linked to the polynucleotide by other means than described above. For example, various bifunctional cross-linking reagents could be used to link amino groups of the HRPO and the polynucleotide, especially N-succinimidyl-3-(2-pyridyldithio)propionate (SPDP) available from Pharmacia Fine Chemicals AB. This alternative method of linking an enzyme directly to the base groups of a single stranded polynucleotide is illustrated by Reaction Scheme B presented hereinafter.

Briefly, in separate steps both the enzyme and the polynucleotide are reacted with the SPDP thereby introducing 3-(2-pyridyldithio)propionyl groups onto $-NH_2$ groups in the enzyme and the polynucleotide. The substituent groups on the polynucleotide are then converted to thiol groups by reaction with dithiothreitol, which thiol groups are condensed in the final step with the substituent groups introduced into the enzyme to provide a bridging group of the formula

$$-\overset{O}{\overset{\|}{C}}-CH_2-CH_2-S-S-CH_2-CH_2-\overset{O}{\overset{\|}{C}}-$$

between amino groups in the enzyme and amino groups in the polynucleotide bases.

The enzymes employed as labels in the present invention are not limited to glycoproteins and could be for example alkaline phosphatase, beta-galactosidase, beta-glucosidase, urease or luciferase. Such enzymes can also be linked to singled strand polynucleotides by covalent bonding, using bonding techniques analogous to those employed in bonding them to antibodies. Bifunctional reagents, e.g. SPDP mentioned above, could be used for this purpose.

For some enzymes, at least, the detection means will very desirably include means for amplifying the enzyme signal or activity. Such means include using the enzyme to trigger a reaction producing detectable fluorescence, e.g. as from alkaline phosphatase and umbelliferyl phosphate, or chemiluminescence, e.g. as from luciferase with luciferin and ATP. Alternatively the enzyme can be used to trigger a reaction or series of reactions, which may include feedback to the primary reaction, giving rise to a detectable product. Such methods are known from European Application 49606 (C.H. Self) and PCT Application WO/8100725 (The Prince Charles Hospital Development Centre Trust, Queensland, Australia).

The marker polynucleotide will normally be DNA or comprise DNA nucleotides and can have any reasonable length, e.g. it can be a fragment produced from a cloning vector by restriction, or a synthetic polynucleotide. In principle it is desirable to use a length of at least 2.5 kilobases, preferably from 5 to 15 kilobases, in order to provide a large number of points of attachment for the enzyme group and therefore a strong signal. The marker polynucleotide must be single-stranded over at least the enzyme-carrying part thereof as base-pairing would not ordinarily permit enzyme group attachment. Conveniently it is single-stranded substantially throughout its length, although it is conceivable that it might have a double-stranded portion at its terminus if it was decided to attach it to a polynucleotide probe, also having a double-stranded portion at a termius, by annealing.

The tails preferably present on the enzyme-labelled polynucleotide and the probe are conveniently poly(dT) and poly(dA), either way around, but could be poly(dC) and poly(dG), either way around. When the enzyme-labelled polynucleotide has a dT tail, and the probe a dA tail, it is suggested that the probe should have a much longer tail than the enzyme-lablled polynucleotide, preferably at least twice as long. Thus, a dA tail of about 200 nucleotides on the probe and a dT tail of about 50 on the enzyme-labelled polynucleotide is suggested. More generally, with a fairly long tail length on the probe it should be possible to hybridise several enzyme-labelled polynucleotides to a single probe molecule, thereby further increasing the strength of the enzyme signal.

The probe polynucleotide contains the desired single-stranded complementary sequence and is conveniently single-stranded over substantially its entire length. The complementary sequence must be long enough to be specific for the sequence to be detected, which means preferably at least 12 nucleotides for most bacterial genomes and at least 14 nucleotides for most human ones even where the sequence to be detected differs from other possible sequences only by a point mutation. Preferably it is up to 19 or 20 nucleotides long. As the tail is preferably at least 20 nucleotides long, the minimum number of polynucleotides in the probe will usually by 12 + 20 = 32 or 14 + 20 = 34.

The method of the invention has an in-built versatility or "universal" nature, because the same enzyme-labelled marker polynucleotide can be used for attachment to a variety of probes specific to a variety of genetic disorders. Thus one can prepare large numbers of probes without having to label them in a series of individual separate operations specific to each. Moreover, it is contemplated that if the tails of the marker polynucleotide are kept reasonably short, more than one molecule thereof can be attached to each molecule of the probe, thus increasing the "concentration" of the enzyme label.

The probe could be prepared from double-stranded DNA identical in sequence to the two strands of a gene sequence to be detected. On denaturation of the ds-DNA, the two strands would then be hybridisable to the two strands of the gene sequence to be detected.

The probing sequence of its ds-precursor can be produced by cloning it in a plasmid or phage to isolate small molecules comprising the desired sequence. Alternatively such molecules could be synthesised chemically or isolated from a polynucleotide of the kind which is to be detected.

The following Examples illustrate the invention.

## EXAMPLE 1

### Preparation of enzyme-labelled single-stranded DNA

STEP A: First 0.8 mg of horseradish peroxidase is oxidised with 0.2 ml of 16 mM sodium periodate for 20 minutes at room temperature. The cis-glycol groups in the sugar side-chains of the glycoprotein are thereby oxidised to aldehydes. The oxidised HRPO is then dialysed overnight, at $4^{o}C$, against 200 ml of 1 mm sodium acetate buffer pH 4.4.

STEP B: Single-stranded phage M13 DNA is elongated using terminal 3'-deoxynucleotidyl transferase, ("TdT"), as described by Deng and Wu, Nucl. Acids Res. 9, 4173, (1981). 70 microlitres of the single-stranded M13 DNA are added to 18 micromoles of dTTP and 3 microlitres of TdT (from P-L Biochemicals) are added. After 2 hours at $30^{o}C$, this produces a tail on average of 70 dT nucleotides attached to the 3'-end of the M13 DNA.

STEP C: 80 micrograms of the poly(dT)-tailed single-stranded phage DNA M13 obtained in Step B are dissolved in 0.2 M sodium bicarbonate buffer, pH 9.5, and the oxidised HRPO obtained in Step A is added; the resultant pH should be between 9 and 9.5, to avoid peroxidase-peroxidase cross-linking. The mixture is stirred for 2 hours at room temperature, during which time

the aldehydes in the HRPO react with the amino groups in the DNA bases to form Schiff bases.

After 2 hours the imine linkages of the Schiffs base are reduced by addition of 20 microlitres of 5 mg/ml sodium borohydride for 2 hours at 40°C. HRPO-DNA conjugates are then separated from free enzyme by gel filtration through a "Biogel A-5M" column, 10 mm x 200 mm. The column was pre-equilibrated and eluted with 0.1 M Sodium phosphate, 0.1 M sodium chloride pH 7.0. By this method at least 40 molecules of HRPO have been coupled to the tailed DNA while retaining a specific activity of 80% of the activity before coupling.

## EXAMPLE 2

The preparation of enzyme-labelled DNA using commercially available N-succinimidyl 3-(2-pyridyldithio) propionate (SPDP) as the cross-linking reagent is illustrated as follows:

STEP A: 4 mg of horseradish peroxidase (HRPO) is dissolved in 2 ml sodium phosphate buffer (0.1 M, pH 7.5) and sodium chloride (0.1 M). A nine fold molar excess of SPDP is added to the stirred HRPO solution and allowed to react for thirty minutes at room temperature. A nine fold molar excess of SPDP to HRPO results in approximately one SPDP molecule substituted per HRPO molecule. Excess SPDP and by-product N-hydroxysuccinimide are removed by gel filtration through a "Sephadex G-25" column (10 mm x 150 mm). Peak fractions containing HRPO substituted with SPDP are pooled. The level of substitution with SPDP can be checked at this stage by taking a 200 microlitre aliquot of the HRPO-SPDP solution and reacting it with dithiothreitol (50 mM final concentration) for twenty minutes at room temperature. The consequent release of the pyridine-2-thione is followed spectrophotometrically by noting an increase in absorbance at 343 nm. Release of pyridine-2-thione is directly proportional to the leve of substitution with SPDP.

STEP B: 100-200 microgram of RAF16 DNA is dissolved in 2 ml sodium phosphate buffer (0.1 M pH 7.5) and sodium chloride (0.1 M). A five thousand fold molar excess of SPDP to DNA is added to the stirred DNA solution and allowed to react for thirty minutes at room temperature. A five thousand fold molar excess of SPDP to DNA results in one SPDP molecule substituted every 20-30 bases. Excess SPDP and by-product N-hydroxysuccinimide are removed by precipitating the SPDP substituted DNA with 2.5 volumes of ethanol and 0.1 volumes of 3 M sodium acetate pH 4.5 at -70°C for thirty minutes. The substituted DNA is washed twice with an equal volume of 70% ethanol, dried under vacuum and finally dissolved in 1 ml of sodium phosphate buffer (0.1 M pH 7.5) and sodium chloride (0.1 M). The 2-pyridyl disulphide groups in the SPDP substituted DNA are then reduced by adding dithiothreitol to a final concentration of 50 mM and incubating at room temperature for twenty minutes. Again the release of pyridine-2-thione can be followed spectrophotometrically and the level of substitution of the DNA by the reduced SPDP can be calculated. Pyridine-2-thione and dithiothreitol are removed from the substituted DNA by precipitating with ethanol at -70°C as described previously. The resultant thiol-subsituted DNA is dissolved in 0.5-1 ml sodium phosphate buffer (0.1 M pH 7.5) and sodium chloride (0.1 M).

STEP C: The thiol containing DNA produced in Step B and the SPDP substituted HRPO obtained in Step A are mixed and incubated at 15°C for 24-36 hours. Again, the reaction and degree of cross-linking can be estimated from the increase in absorbance at 343 nm due to the release of pyridine-2-thione. The HRPO-DNA conjugate is separated from free enzyme by gel filtration through a "Biogel A-5M" column (150 mm x 220 mm). The column is pre-equilibrated and eluted with sodium phosphate buffer (0.1 M pH 7.5) and sodium chloride (0.1 M).

In a final step the HRPO-DNA conjugate in obtained in Step C is elongated with a poly(dA) or poly(dT) tail using terminal 3'-deoxynucleotidyl transferase (TdT) as in Example 1. In an alternative procedure, particularly when a poly(dT) tail is used, the DNA can be tailed before the reaction with the SPDP as in Step B.

## EXAMPLE 3

### Preparation of enzyme-labelled poly(dA)-tailed polynucleotide

The procedure of Example 1 is repeated except that 18 micromoles of dATP are used instead of the dTTP in Step B and that Steps B and C are carried out in reverse order, that is to say the oxidised HRPO produced in Step A is reacted wih the single-stranded polynucleotide M13 DNA to form a HRPO/M13 DNA conjugate before attachment of the poly(dA) tail. This reverse order is necessary to ensure attachment of the HRPO to the base groups of the M13 DNA and leaving the base groups of the poly(dA) tail free for subsequent hybridisation with a poly(dT)-tailed polynucleotide probe. In this Example incubation of the 18 micromoles dATP with the HRPO/M13 DNA conjugate in the presence of the terminal transferase (TdT) at 30$^{o}$C for 30-40 minutes produces a poly(dA) tail of approximately 270 dA nucleotide bases attached to the 3'-end of the HRPO/M13 DNA conjugate.

## EXAMPLE 4

### Preparation of the polynucleotide probe

For experimental purposes the DNA to be detected is assumed to be a sequence complementary to a sequence present in linearised DNA obtained from the well-known E. coli plasmid pAT 153. This sequence can be regarded for the purposes of simple illustration as analoguous to a "W" sequence occurring in the beta-globin genes. Accordingly pAT 153 can be regarded as containing a sequence analoguous to "c W", i.e. the complementary sequence capable of hybridisation to W through base-pairing.

To 5 micrograms of the plasmid pAT 153, which is commercially available, in 20 microlitres of 50 mM Tris HCl at pH 8, 10 mM magnesium chloride, 50 mM sodium chloride and 200 micrograms per ml of bovine serine albumin, are added 5 units of Pst I. Pst I cleaves pAT 153 at a single

restriction site, producing a double-stranded linear DNA molecule of just under 4,000 bp in length. The restriction is carried out at 37°C for 1 hour and the DNA is purified by precipitation from ethanol as described above. The ds-DNA is tailed using 3'-TdT, as described above, Example 1 Step B except that dATP (18 micromoles) is used in place of dTTP. After 2 hours at 30°C the mean tail length is 200 dA nucleotides. The ds-pAT 153 - poly(dA)-tailed probe DNA is then electrophoresed through a 1% agarose gel at 50 V for 12 hours and the gel is treated with alkali to denature the DNA which is then transferred by Southern blotting onto cellulose nitrate filters. These are then baked at 80°C for 2 hours to immobilize the ss-DNA. Amounts of ss-DNA from 250 nangrams down to 1.5 picograms, i.e. down to a minimum of 0.64 attomoles of the poly(dA)-tailed plasmid pAT 153 DNA, are thus applied and immobilized onto the cellulose nitrate filters.

## EXAMPLE 5

Example 4 is repeated except that 18 micromoles of dTTP are used in place of dATP to produce a single-stranded poly(dT)-tailed pAT 153 DNA probe.

## EXAMPLE 6

### Hybridisation of enzyme-labelled polynucleotide and the polynucleotide probe

The poly(dA)-tailed pAT 153 DNA-containing filters obtained in Example 4 were prehybridised as follows: the filters were first washed with an aqueous 0.45 M NaCl, 0.06 M sodium citrate solution at pH 7.5 (3xSSC) at 37°C for 30 minutes, and then for 3 hours at 40°C with 3xSSC additionally containing 0.1% sodium dodecyl sulphate (SDS), approximately 2-5 ml. Denhardts reagent (0.2% Ficol, 0.2% polyvinylpyrrolidione, 0.2% bovine serum albumin), and 50 microgrammes per ml of denatured herring sperm DNA.

After prehybridisation the filters were transferred to 50 ml of the latter solution additionally containing also 0.1 microgram/ml of the M13-dT-tailed-HRPO reagent produced in Example 1. Hybridisation was carried out at 40°C for 3 hours. The filters were then washed four times, 5 minutes each, with 2xSSC (an aqueous solution containing 0.3 M sodium chloride, 0.04 M trisodium citrate) and 0.1% SDS at room temperature.

The filters were allowed to dry and then stained using 2.5 mM tetramethyl benzidine and 0.75 mM $H_2O_2$, in 0.05 M HCl. Within 15 minutes, spots were clearly visible on all places where pAT 153 DNA has been applied indicating hybridisation of the enzyme-labelled polynucleotide to the poly(dA)-tailed pAT 153 DNA. The enzyme-labelled polynucleotide had 300 residues of HRPO per M13-poly T molecule. Hence the maximum amount of HRPO which could have hybridised to 0.64 attomoles of dA-tailed pAT 153 would be 192 attomoles.

Quantitative tests have established that 250 attomoles of HRPO will give sufficient colour intensity to be visible to the naked eye within 5 minutes. Since at least 40 molecules of HRPO can be linked to one molecule of M13, this implies that the method can detect 8 attomoles of M13 (assuming the HRPO molecules bound to the M13 are 80% active).

In a repeat of the above procedures, similar results were obtained using the poly(dT)-tailed pAT 153 DNA probe of Example 5 and the HRPO-labelled, poly(dA)-tailed M13 DNA of Example 3.

Protocol for a diagnostic method in accordance with the invention

10 micrograms of sample DNA, for example, from a human patient and containing a nucleotide sequence to be detected are digested with appropriate restriction endonuclease and electrophoresed through agarose gel. The DNA fragments are transferred to nitrocellulose by "blotting"; hybridised (at 65°C) to an excess of a poly(dA)- or poly(dT)-tailed ss-polynucleotide probe containing the complementary sequence. The

excess probe is removed by washing. The resultant duplex is then hybridised via its poly(dA) or (dT)-tail to excess HRPO-M13-poly(dT)-tailed reagent (Example 1) or HRPO-M13-poly(dA)-tailed reagent (Example 3), as appropriate, at 40°C. The DNA is thereby labelled with the enzyme. The excess HRPO-labelled reagent is then removed from the product by washing and the presence, or absence, of the enzyme label on the sample detected as described above.

This protocol, i.e. involving hybridisation of the probe to the sample, prior to the hybridisation of the enzyme-labelled polynucleotide to the probe is preferred because the hybridisation of the probe to the sample usually requires stringent conditions, notably a high temperature, which may no necessarily be withstood by the enzyme label. Accordingly, it is preferred to attach the probe to the sample, and then hybridise the enzyme label to the probe usually under less severe conditions which will be more easily withstood by the enzyme.

Whilst the methods particularly described herein utilise HRPO as the enzyme label, and the subsequent detection of any enzyme associated activity in the labelled sample by staining using tetramethyl benzidine and hydrogen peroxide, other enzyme labels may be used and other detection methods used without departing from the scope of the invention as claimed.

REACTION SCHEME B

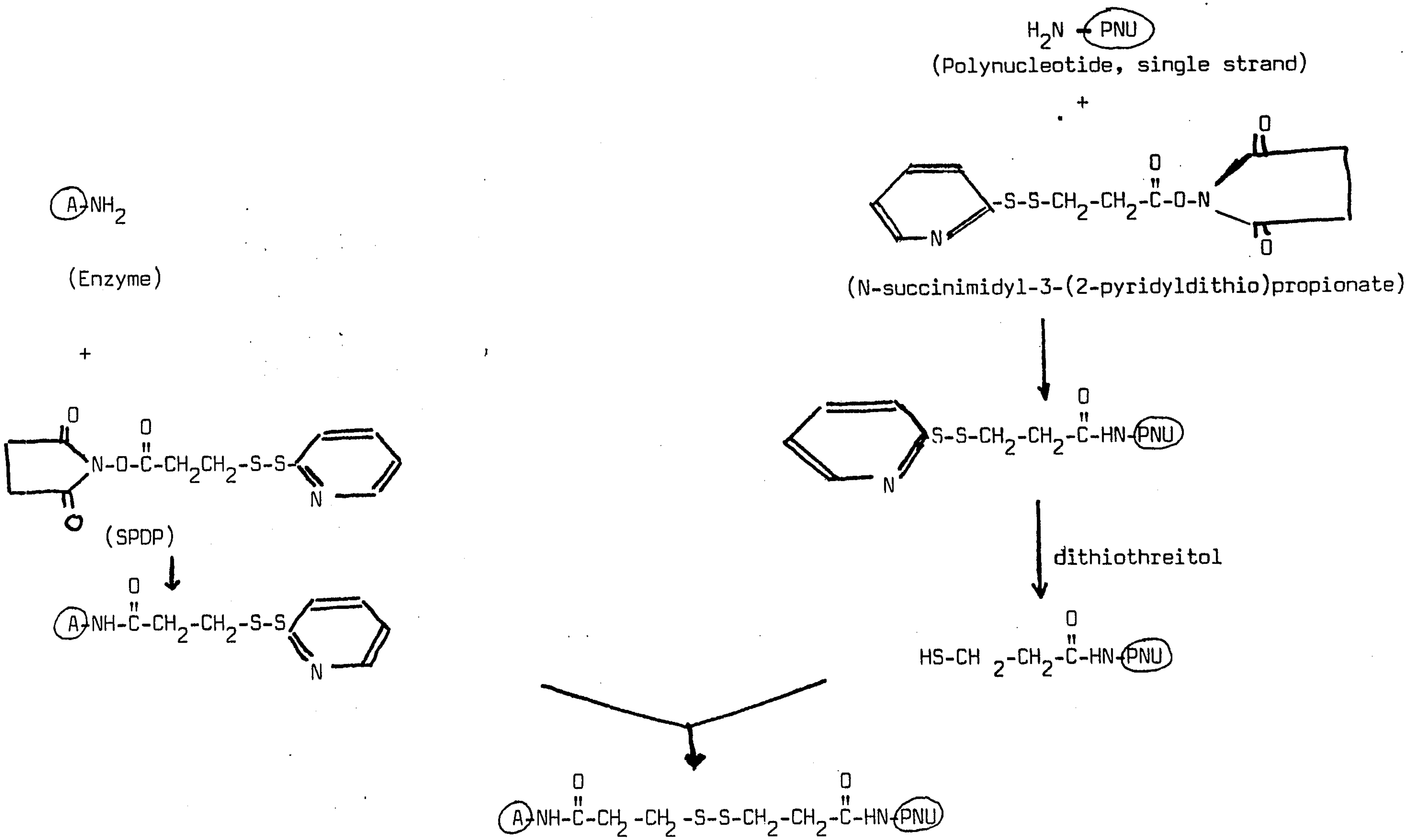


Enzyme/PNU conjugate

## CLAIMS

1. A method for the detection of a given nucleotide sequence in a polynucleotide, which comprises contacting the polynucleotide under the hybridisation conditions with a polynucleotide probe comprising a single-stranded nucleotide sequence complementary to the sequence to be detected and a reactive tail capable of undergoing coupling with an enzyme-labelled marker, coupling said marker to said tail, before or after hybridisation of the probe with said polynucleotide, assuming the presence therein of the given sequence, and determining the presence, or absence, of enzyme-associated activity in the sample after the said hybridisation with the probe and the said coupling of the marker to the probe, wherein the marker comprises a polynucleotide having a single-stranded portion to which the enzyme is attached by means of chemical linkages to one or more of the base groups in said single-stranded portion and a polynucleotide tail, the said probe likewise comprising a polynucleotide tail, the reactive tails of the probe and the marker both comprising nucleotide sequences differing from said complementary sequence and from the sequence to be detected, and wherein the probe is coupled to the marker by coupling the polynucleotide tail of the probe to the polynucleotide tail of the marker.

2. A method according to claim 1, wherein the polynucleotide tails of the probe and the marker are both single-stranded nucleotide sequences complementary one to the other, but neither being complementary to the sequence to be detected, and wherein the coupling of the probe to the marker is effected by hybridisation of the tail of the marker to the tail of the probe.

3. A method according to claim 2, wherein the polynucleotide tails of the probe and the marker are both homogeneous nucleotide sequences complementary one to the other.

4. A method according to claim 3, wherein one of the polynucleotide tails is a poly(dA) tail and the other is a poly(dT) tail.

5. A method according to any one of claims 1-4, wherein the marker comprises a glycoproteinaceous enzyme directly linked to the base groups of the single-stranded portion of the polynucleotide by means of chemical coupling between the glycollic groups of the glycoprotein to the amine groups of the nucleotide base groups.

6. A method according to claim 5, wherein the enzyme is horseradish peroxidase.

7. A method according to claim 6, wherein the marker is HRPO labelled, single-stranded, poly(dA) or poly(dT)-tailed phage M13 DNA.

8. An enzyme-containing polynucleotide marker comprising a single-stranded polynucleotide sequence having one or more enzymatically active groups chemically linked to the base groups of said single-stranded polynucleotide sequence.

9. A marker according to claim 8 further comprising a polynucleotide tail.

10. A marker according to claim 9, wherein said tail is a single-stranded polynucleotide tail capable of hybridisation with another polynucleotide possessing a single-stranded nucleotide sequence complementary to that of said tail.

11. A marker according to claim 10, wherein the polynucleotide tail consists of or comprises a homogeneous polynucleic acid sequence.

12. A marker according to claim 11, wherein the polynucleotide tail is a poly(dA) or poly(dT) tail.

13. A marker according to any one of claims 8-12, wherein the enzymatically active groups is or are derived from a glycoproteinaceous enzyme, and each group is attached to the single-stranded polynucleotide sequence by chemical bonds between glycollic groups in the glycoprotein and amine groups in the base groups of said polynucleotide sequence.

14. A marker according to any one of claims 8-13, wherein the enzymatically active groups is or are horseradish peroxidase groups.

15. HRPO-labelled, single-stranded, poly(dA) or poly(dT)-tailed phage M13 DNA.

16. A kit for the detection of a given nucleotide sequence in a polynucleotide sample comprising, as a first component, a polynucleotide probe comprising a single-stranded nucleotide sequence complementary to the sequence to be detected and a polynucleotide tail, and as a second component a marker comprising a polynucleotide having a single-stranded portion to the base groups of which is or are attached one or more enzymatically active groups, and a polynucleotide tail, the two tails being capable of coupling one to the other before or after hybridisation of the probe with the sample.

17. A kit according to claim 16, wherein both the probe and the marker have single-stranded polynucleotide tails, the tail on the probe being complementary to that on the marker.

18. A kit according to claim 16 or 17, wherein the marker is an enzymatic marker as claimed in any one of claims 11-15.

0124221

European Patent Office

# EUROPEAN SEARCH REPORT

Application number

EP 84 30 1601

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | GB-A-2 019 408 (INSTITUT PASTEUR)<br>* Whole document * | 1 | C 12 Q 1/68 |
| X | EP-A-0 070 687 (STANDARD OIL COMPANY)<br>* Pages 6,7; page 8, lines 1-15; claims 1-4 * | 1,6,7, 13,14 | |
| X | EP-A-0 070 685 (STANDARD OIL COMPANY)<br>* Whole document * | 1,6,7, 13,14 | |
| A | CHEMICAL ABSTRACTS, vol. 91, no. 23, 3rd December 1979, page 219, no. 188423w, Columbus, Ohio, USA; M. SUGIURA et al.: "Methods for enzymic labeling of nucleic acids and polynucleotides" & SEIKAGAKU JIKKEN KOZA 1977, 6(TORESA JIKKENHO, Pt. 2), 515-525<br>* Abstract * | | TECHNICAL FIELDS SEARCHED (Int. Cl. 3)<br>C 12 Q<br>C 07 H |

The present search report has been drawn up for all claims

| Place of search | Date of completion of the search | Examiner |
|---|---|---|
| THE HAGUE | 27-07-1984 | MEYLAERTS H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document